# EUROPEAN PATENT APPLICATION

(11) **EP 4 112 742 A1**
(43) Date of publication of application: **04.01.2023**
(21) Application number: 21182481.8
(22) Date of filing: 29.06.2021
(51) Int. Cl.: C12Q 1/6806

(54) **METHOD FOR GENERATING REGION-SPECIFIC AMPLIFICATION TEMPLATES**

(71) Applicant: Leica Microsystems CMS GmbH, 35578 Wetzlar (DE)
(72) Inventor: Falk, Schlaudraff,, 35510 Butzbach (DE)
(74) Representative: Schaumburg und Partner Patentanwälte mbB

(57) **Abstract**

A method for generating region-specific amplification templates (102, 204) of a biological sample (400) is provided, comprising the following steps: adding at least a first plurality of oligonucleotide constructs (100, 200) comprising a first cage molecule (112) and a second plurality of oligonucleotide constructs (100, 200) comprising a second cage molecule (112) to the biological sample (400); synthesising a complementary first strand (118) from a template (116) bound to target binding regions (110) of the oligonucleotide constructs (100, 200); scanning at least a first region of interest (406, 408) of the biological sample (400) with a first focused light beam and a second region of interest (406, 408) of the biological sample (400) with a second focused light beam to form uncaged first oligonucleotide constructs (120) in the first region of interest (406, 408) and uncaged second oligonucleotide constructs (120) in the second region of interest (406, 408); and synthesising a complementary second strand (122) to form amplification templates (102, 204). In a further aspect a system for performing the method is provided.

## Description

### Technical field

The invention relates to a method for generating region-specific amplification templates of a biological sample. In a further aspect a system for performing the method is disclosed.

### Background

Transcriptomics technologies enable the study of an organism's transcriptome, that is, of all the transcripts present in a biological sample. The study of transcriptomes is nowadays an important way to understand how a single genome may give rise to different cell types and how gene expression is regulated. Further, transcriptomics analysis enables better understanding of disease development and progression, for example, through changes in the transcripts of cancerous cells or in host-pathogen interactions. Importantly, for cell or tissue type level analysis a high resolution is required to enable discriminating between these cell types, tissue types or other functionally similar structures. A range of transcriptomics methods have been developed to enable this.

High throughput methods such as fluorescence activated cell sorting or microfluidic devices enable isolating a large number of cells for single cells analysis. With subsequent transcriptomics analysis, this requires dissolution of a tissue sample in the process of which information about the spatial arrangement of the cells in the original tissue sample and their identity may get lost.

Laser Micro-Dissection, as described in the document DE 100 18 253 C2, enables separating and removing regions of interest from a biological sample. The removed regions of interest may subsequently be analysed by transcriptomics methods. However, this method is labour intensive with a requirement for the complex handling of a large number of microscopic samples.

The documents WO 2020/235563 A1 and Honda et al. 2020 disclose a transcriptomics method for determining the transcriptome of a region of interest of a biological sample based on photo-caged oligodeoxynucleotides (Honda M, Shinya O, Harada A, Maehara K, Tanaka K, Meno C, Ohkawa Y. High resolution spatial transcriptome analysis by photo-isolation chemistry. bioRxiv, 2020.03.20.000984). However, only a single region of interest can be analysed by the method in the disclosed documents, such that analysis results can be assigned with certainty to the single region of interest. In case of several regions of interest, the analysis results cannot be assigned to their respective original region of interest. Hence, the determination of transcriptomes for at least several regions of interest and their separate and individual analysis is not possible.

### Summary

It is an object to provide a method for generating region-specific amplification templates of a biological sample and a system for performing the method, which enable generating region-specific amplification templates for at least two regions of interest with high spatial resolution.

The aforementioned object is achieved by the subject-matter of the independent claims. Advantageous embodiments are defined in the dependent claims and the following description.

A method for generating region-specific amplification templates of a biological sample is provided, comprising the following steps: adding at least a first plurality of oligonucleotide constructs and a second plurality of oligonucleotide constructs to the biological sample, wherein the oligonucleotide constructs each comprise a promoter region, an adapter region, a target binding region, wherein the first oligonucleotide constructs each further comprise a first identifying region and a first photoremovable cage molecule, and wherein the second oligonucleotide constructs each further comprise a second identifying region and a second photoremovable cage molecule; synthesising a complementary first strand from a template bound to the target binding regions of the oligonucleotide constructs; scanning at least a first region of interest of the biological sample with a first focused light beam and a second region of interest of the biological sample with a second focused light beam to form uncaged first oligonucleotide constructs in the first region of interest and uncaged second oligonucleotide constructs in the second region of interest; and synthesising a complementary second strand from the uncaged oligonucleotide constructs to form first amplification templates originating from the first region of interest and second amplification templates originating from the second region of interest. This enables capturing the genetic content, in particular the transcriptome, in several regions of interest of a biological sample, for example a tissue section, with high spatial resolution and whilst maintaining the ability to assign each of the generated amplification templates to a specific one of the regions of interest. Thus, the generated amplification templates are assigned to the respective region of interest. In other words, each amplification template is assigned to the region of interest the respective amplification template originated from. The ability to assign the amplification templates to specific regions of interest also enables the comparison of amplification templates from these specific regions of interest to each other. For example, the amplification templates of a region of interest with cancerous cells may be compared to the amplification templates of a region of interest with healthy cells.

In a preferred embodiment, the first light beam and the second light beam have differing light parameters. Light parameters may be the wavelength of the light, the intensity of the light or the duration of scanning of a particular point in the region of interest. In particular, the values of these parameters are different for the different light beams when scanning the regions of interest of the biological sample. This enables robust photoremoval of either the first cage molecule or the second cage molecule and therefore assignment of the first amplification template to the first region of interest and the second amplification template to the second region of interest. Further, in particular, the light may have a wavelength longer than ultraviolet light, for example, in the near-infrared light range (700 - 1400 nm). This enables scanning of the biological sample with less damage to biological structures in the biological sample compared to UV light.

In particular, the first cage molecule is photoremovable from the first oligonucleotide construct only by the first focused light beam, and the second cage molecule is photoremovable from the second oligonucleotide construct only by the second focused light beam. This enables particularly robust photoremoval of either the first cage molecule or the second cage molecule and therefore assignment of the first amplification template to the first region of interest and the second amplification template to the second region of interest.

Preferably, the uncaged oligonucleotide constructs, or the amplification templates are extracted from the biological sample. This requires prior lysis of the sample. This enables further analysis of the amplification templates.

In a particularly preferred embodiment, the first oligonucleotide construct further comprises a first affinity reagent and the second oligonucleotide construct further comprises a second affinity reagent. The affinity reagent may an antibody, an aptamer or a Fab fragment, for example. The first and second affinity reagents have different affinities from each other. This enables identifying the oligonucleotide constructs by affinity binding.

Preferably, the first photoremovable cage molecule is bound to the first affinity reagent and the second photoremovable cage molecule is bound to the second affinity reagent. In particular, the affinity reagent is linked to the backbone of the oligonucleotide constructs. This enables selectively uncaging the affinity reagents.

Preferably, the uncaged oligonucleotide construct or the amplification templates are extracted selectively by affinity capture based on their respective affinity reagent. The affinity capture or binding can be performed by affinity chromatography, for example. This enables separation or extraction of amplification templates from regions of interest and the biological sample. Alternatively, this enables separation or extraction of uncaged oligonucleotide constructs.

Preferably, the target binding region is a poly-T region. This means the target binding region is comprised of thymine residues. The poly-T region 110 consists of between 5 and 50 thymine nucleotides, in particular between 8 and 30 thymine nucleotides, preferably between 10 to 20 thymine nucleotides. This enables hybridising the oligonucleotide constructs to mRNA molecules, for example, of the transcriptome of the cells of the biological sample.

In a preferred embodiment, the amplification templates are amplified with a polymerase specific to the promoter regions of the oligonucleotide constructs. This may be, for example, by polymerase chain reaction (PCR) or in vitro transcription. This enables further analysis of the amplification templates.

Preferably, the promoter region is a T7 promoter and the polymerase is a T7 polymerase. This enables particularly fast amplification of the amplification templates.

Preferably the amplified amplification templates are sequenced. The sequence information includes the sequence of the identifying region of the amplification templates. This enables assignment of each of the amplification templates and their sequence information to a specific one of the regions of interest. Alternatively, the amplification templates may have been separated by affinity capture to enable assignment to a specific one of the regions of interest.

Preferably, the regions of interest are scanned by means of an optical scanning unit comprising two prisms arranged in the beam path of the first light beam and the second light beam. This enables directing the scanning light beams with a particularly high spatial resolution. Alternatively or in addition, the scanning unit comprises a piezo-based element or an acousto-optic modulator for guiding or directing the scanning light beams.

In a particularly preferred embodiment, the biological sample is stained and imaged prior to directing the light beams to the regions of interest, or the biological sample is one of a plurality of sections and wherein one of the sections adjacent to the biological sample is stained and imaged prior to directing the light beams to the region of interests. For example, the biological sample may be biopsy, with the sections being subsequent sections of the biopsy. This enables precise determination of the regions of interest.

Preferably, at least the first and the second regions of interest of the biological sample are identified in the image data and respective coordinates of a scanning coordinate system are generated to scan the respective coordinates of at least the first and second regions of interest with the respective light beam. This enables a precise scanning of the regions of interest.

In a further aspect a system is provided for performing the method for generating region-specific amplification templates, the system comprising: an imaging unit configured to image a biological sample; a scanning unit configured to scan at least a first and a second region of the biological sample with a respective first or second focused light beam. The scanning unit may comprise a LED light source to generate the light beams. This enables capturing the genetic content, in particular the transcriptome, in several regions of interest of a biological sample, for example a tissue section, with high spatial resolution and whilst maintaining the ability to assign the generated amplification templates to a specific one of the regions of interest.

Preferably, the scanning unit is configured to generate the first light beam and the second light beam with differing light parameters. This enables robust photoremoval of either the first cage molecule or the second cage molecule.

Preferably, the scanning unit comprises two prisms arranged in the beam path of the first and second light beam. For example, the document DE 100 18 253 C2 discloses a suitable arrangement and control of prisms. This enables precisely directing the scanning light beams and with a particularly high spatial resolution. Alternatively or in addition, the scanning unit comprises a piezo-based element or an acousto-optic modulator for guiding or directing the scanning light beams.

### Short Description of the Figures

Hereinafter, specific embodiments are described referring to the drawings, wherein:
- Fig. 1: shows a schematic view of an oligonucleotide construct and a flow chart for generating a corresponding amplification template,
- Fig. 2: shows a schematic view of an oligonucleotide construct with an affinity reagent and a flow chart for generating a corresponding amplification template,
- Fig. 3: shows a flow chart for a method for generating region-specific amplification templates of a biological sample, and
- Fig. 4: shows a schematic view of a biological sample.

### Detailed Description

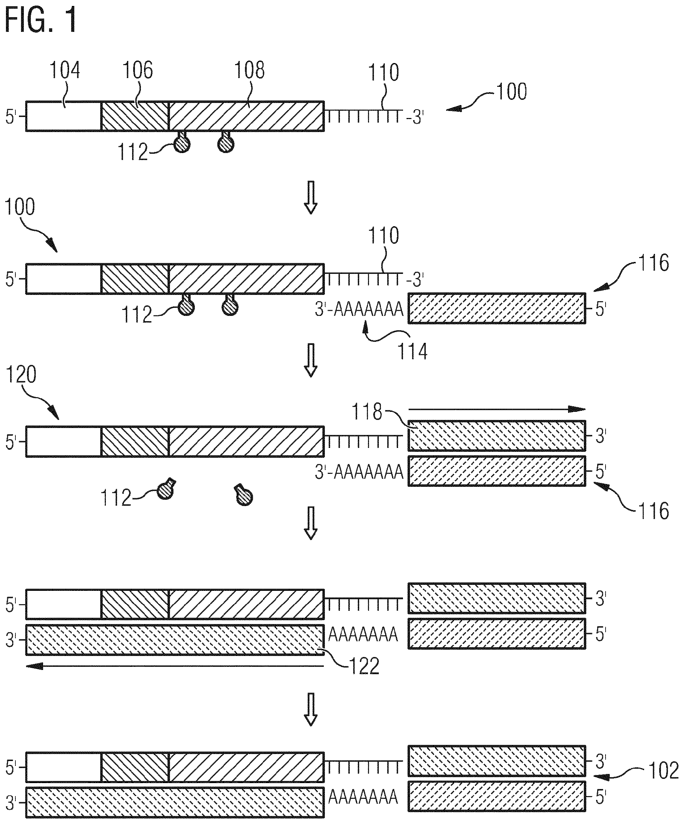

Figure 1 shows a schematic view of an oligonucleotide construct 100 and a flow chart for generating a corresponding amplification template 102. The oligonucleotide construct 100 is a single stranded deoxyribonucleic acid (DNA) molecule comprising a promoter region 104, an identifying region 106, an adapter region 108, and a target binding region 110. The target binding region 110 is, in particular, a poly-thymine (poly-T) region 110. The poly-T region 110 consists of between 5 and 50 thymine nucleotides, in particular between 8 and 30 thymine nucleotides, preferably between 10 to 20 thymine nucleotides. The promoter region 104 is at a 5' end of the oligonucleotide construct 100 and the binding region 110 is at a 3' end of the oligonucleotide construct 100. Moreover, the oligonucleotide construct 100 comprises at least one photoremovable cage molecule 112. The photoremovable cage molecule 112 is a photolabile protecting group and cleavable from the oligonucleotide construct 100 by light at a predetermined wavelength. In particular, the cage molecule 112 may be covalently bound to a nucleobase of the adapter region 108, with the light causing the covalent bond to break and release the cage molecule. The bound cage molecule 112 prevents, for example, the binding of enzymes to the promoter region 104. The bound cage molecule 112 further prevents hybridisation particularly of the regions 104, 106, 108 to a complementary DNA strand. Regularly, the construct 100 comprises a plurality of cage molecules 112.

The adapter region may be a random sequence of nucleic acids. In particular, the adapter region may be between 5 and 100 nucleotides long, preferably between 10 and 25 nucleotides long.

The identifying region 106 comprises a unique code to identify the oligonucleotide construct 100. The unique code is a short sequence of nucleic acids. In particular, a sequence between 3 and 6 nucleic acids long, the nucleic acids being adenine, thymine, guanin and cytosine, for example.

The poly-T region 110 of the oligonucleotide construct 100 may bind a target nucleic acid sequence in a biological sample. For example, in case the oligonucleotide construct 100 is added to a permeabilised tissue section, the oligonucleotide construct 100 may enter the permeabilised cells of the tissue section and hybridise to the target nucleic acid sequence. In case of the poly-T region 110, the target nucleic acid sequence is a poly-adenosine (poly-A) sequence 114. The poly-A sequence 114 may be part of a single stranded ribonucleic acid such as a messenger RNA (mRNA) 116. Thus, the oligonucleotide construct 100 hybridises to the mRNA 116, in particular, the poly-T region 110 of the construct 100 hybridises to the poly-A sequence 114 of the mRNA 116.

Alternatively, the target binding region 110 of the oligonucleotide construct 100 may comprise a different sequence of nucleic acids and bind to a complementary different target nucleic acid sequence in the biological sample.

With the mRNA 116 bound to the construct 100, a complementary first strand 118 may be synthesised from the mRNA 116. The first strand 118 may be synthesised by an enzyme such as a reverse transcriptase. The enzyme may be added to the oligonucleotide construct 100 in the permeabilised tissue section together with additional elements required for synthesis of the first strand 118 such as nucleotides. In particular, the complementary first strand 118 is a DNA strand complementary to the mRNA 116.

Further, the photoremovable cage molecule 112 may be removed from the oligonucleotide construct 100 by light to give an uncaged oligonucleotide construct 120. The uncaged oligonucleotide construct 120 enables synthesis of a complementary second strand 122 by an enzyme such as a DNA polymerase. The enzyme may be added to the uncaged oligonucleotide construct 120 together with additional elements required for synthesis of the second strand 122 such as nucleotides. In particular, the complementary second strand 122 is a DNA strand complementary to the promoter region 104, the identifying region 106 and the adapter region 108. The second strand 122 cannot be synthesised on oligonucleotide constructs 100 that remain caged by the cage molecule 112.

The uncaged oligonucleotide construct 120 combined with the mRNA 116, the complementary first strand 118 and the complementary second strand 122 form the amplification template 102. The amplification template 102 may be amplified by a polymerase specific to the promoter region 104 of the oligonucleotide construct 100. For example, the polymerase may be a T7 polymerase. Regularly, this is achieved by performing a polymerase chain reaction (PCR) on the amplification template 102. Alternatively, in-vitro transcription may be used to amplify the amplification template 102. The amplified amplification template 102 may then be sequenced in order to identify the sequence of nucleic acids of the amplification template 102. In particular, sequencing may be used to identify the sequence of nucleic acids of the mRNA 116 and/or the complementary first strand 118.

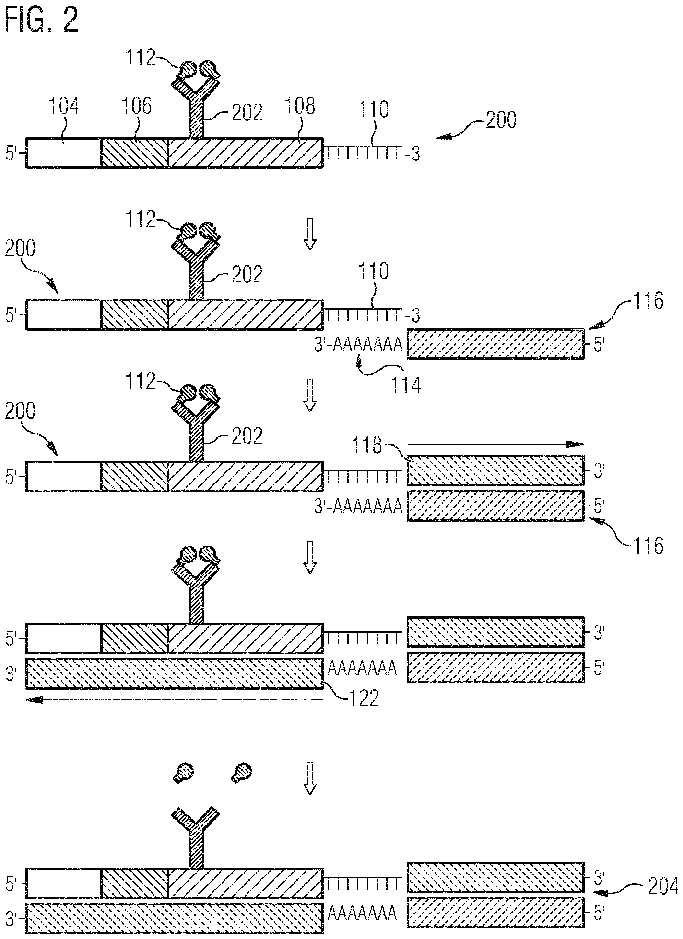

Figure 2 shows a schematic view of an oligonucleotide construct 200 with an affinity reagent 202 and a flow chart for generating a corresponding amplification template 204. Functionally similar elements are marked with the same reference signs.

The oligonucleotide construct 200 is a single stranded deoxyribonucleic acid (DNA) molecule comprising the promoter region 104, the identifying region 106, the adapter region 108, and the target binding region 110. The target binding region 110 is, in particular, a poly-thymine (poly-T) region 110. The poly-T region 110 consists of between 5 and 50 thymine nucleotides, in particular between 8 and 30 thymine nucleotides, preferably between 10 to 20 thymine nucleotides. The promoter region 104 is at a 5' end of the oligonucleotide construct 200 and the target binding region 110 is at a 3' end of the oligonucleotide construct 200.

The affinity reagent 202 binds to another substance with high specificity and can subsequently be released from the substance. The affinity reagent 202 may be used to bind the affinity reagent 202 with the oligonucleotide construct 200 and selectively remove it from a mixture, for example by affinity chromatography. The affinity reagent 202 is, for example, an antibody 202. The antibody 202 may be covalently bound with its Fc region to the phosphoribose backbone of the oligonucleotide construct 200, in particular the adapter region 108. Alternatively, the affinity reagent 202 may be an aptamer or a Fab fragment.

Moreover, the oligonucleotide construct 200 comprises at least one of the photoremovable cage molecule 112. The photoremovable cage molecule 112 is a protective group and cleavable from the oligonucleotide construct 200 by light at a predetermined wavelength. The cage molecule 112 is bound to the antibody 202 such that the cage molecule blocks the binding of the antibody 202. In particular, the cage molecule 112 may be covalently bound to the Fab region of the antibody 202, with the light causing the covalent bond to break and release the cage molecule. Regularly, the construct 100 comprises a plurality of cage molecules 112 bound to the antibody 202.

The identifying region 106 comprises a unique code to identify the oligonucleotide construct 200. The unique code is a short sequence of nucleic acids. In particular, a sequence between 3 and 6 nucleic acids long, the nucleic acids being adenine, thymine, guanin and cytosine, for example.

The poly-T region 110 of the oligonucleotide construct 200 may bind a target nucleic acid sequence in a biological sample, similarly as described for the oligonucleotide construct 100. In case of the poly-T region 110, the target nucleic acid sequence is the poly-adenosine (poly-A) sequence 114, for example, of the messenger RNA (mRNA) 116. Thus, the oligonucleotide construct 200 hybridises to the mRNA 116, in particular, the poly-T region 110 of the construct 200 hybridises to the poly-A sequence 114 of the mRNA 116.

Alternatively, the target binding region 110 of the oligonucleotide construct 200 may comprise a different sequence of nucleic acids and bind to a different target nucleic acid sequence in the biological sample.

With the mRNA 116 bound to the construct 200, the complementary first strand 118 may be synthesised from the mRNA 116. The first strand 118 may be synthesised by an enzyme such as a reverse transcriptase. The enzyme may be added to the oligonucleotide construct 200 in the permeabilised tissue section together with additional elements required for synthesis of the first strand 118 such as nucleotides. In particular, the complementary first strand 118 is a DNA strand complementary to the mRNA 116.

Further, the complementary second strand 122 may be synthesised from the oligonucleotide construct 200 by an enzyme such as a DNA polymerase. The enzyme may be added to the oligonucleotide construct 200 together with additional elements required for synthesis of the second strand 122 such as nucleotides. In particular, the complementary second strand 122 is a DNA strand complementary to the promoter region 104, the identifying region 106 and the adapter region 108.

The uncaged oligonucleotide construct 200 combined with the mRNA 116, the complementary first strand 118 and the complementary second strand 122 form the amplification template 204. The amplification template 204 may be amplified by a polymerase specific to the promoter region 104 of the oligonucleotide construct 200. Regularly, this is achieved by performing a polymerase chain reaction (PCR) on the amplification template 204. Alternatively, in-vitro transcription may be used to amplify the amplification template 204. The amplified amplification template 204 may then be sequenced in order to identify the sequence of nucleic acids of the amplification template 204. In particular, sequencing may be used to identify the sequence of nucleic acids of the mRNA 116 and/or the complementary first strand 118.

Further, the photoremovable cage molecule 112 may be removed. In Figure 2, the photoremovable cage molecules 112 are removed from the affinity reagent 202 of the amplification template 204 this enables selective extraction of the amplification template 204 from the biological sample, in particular, prior to amplifying the amplification template 204. Alternatively, the cage molecules 112 may be removed at a different stage, for example, from the oligonucleotide construct 200 with the mRNA 116 bound to the oligonucleotide construct 200. This would enable extraction of the oligonucleotide construct 200 with the mRNA 116 by the affinity mechanism prior to synthesising the first and second strand 118, 122.

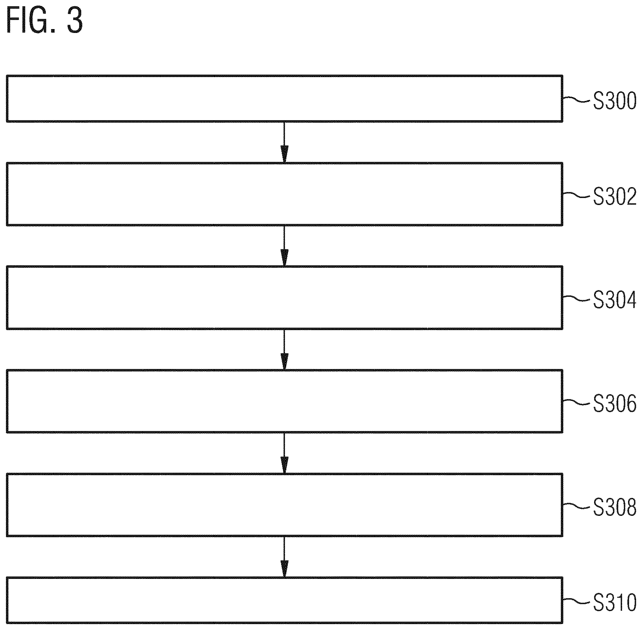

Figure 3 shows a flow chart for a method for generating region-specific amplification templates of a biological sample.

The method starts with step S300. In step S302 at least a first plurality of oligonucleotide constructs and a second plurality of oligonucleotide constructs are added to a biological sample, such as a tissue section. The biological sample is treated such that the first and second plurality of oligonucleotide constructs can pass cell membranes and enter the cytoplasm of cells of the biological sample. For example, the biological sample is permeabilised.

The first and second oligonucleotide constructs are the oligonucleotide constructs 100, 200 described in connection with Figures 1 and 2, for example. Thus, they comprise the promoter region 104, the adapter region 108 and the target binding region 110.

However, the first oligonucleotide construct and the second oligonucleotide construct differ from each other in their identifying regions 106 and their bound photoremovable cage molecules 112. Specifically, the plurality of first oligonucleotide constructs comprise a first identifying region with a first unique sequence and the plurality of second oligonucleotide constructs comprise a second identifying region with a second unique sequence. This enables identifying the respective originating first or second oligonucleotide constructs when sequencing the amplified amplification templates.

Further, the plurality of first oligonucleotide constructs comprise at least a first photoremovable cage molecule and the plurality of second oligonucleotide constructs comprise at least a second photoremovable cage molecule. The first and second cage molecule differ in their conditions under which they are photoremovable from the respective oligonucleotide construct. Specifically, the first and second cage molecule may be removed from the respective oligonucleotide construct by light with differing parameters. These parameters include wavelength, intensity and duration of scanning. For example, the first cage molecule may be a protecting group removeable by ultraviolet light such as 6-nitropiperonyloxymethyl. Whereas the second cage molecule may be a protecting group removable by a different wavelength, for example, near-infrared light, as described by Vorobev and Moskalensky (c.f. Vorobev AY, Moskalensky AE. Long-wavelength photoremovable protecting groups: On the way to in vivo application. Computational and Structural Biotechnology Journal, 2020, 18, 27-34.). This enables selectively removing the first or second cage molecules from the respective plurality of oligonucleotide constructs. In addition, scanning with longer wavelength light, such as near-infrared light, results in less damage of the biological structures scanned by the light beam.

Upon addition of the first and second plurality of oligonucleotide constructs to the biological sample, the target binding regions hybridise to mRNA molecules 116 of the cells of the biological sample. The mRNA molecules 116 of the cells represent the transcriptome of the respective cell.

In step S304 the complementary first strand 118 is synthesised from the mRNA molecules 116 bound to the pluralities of oligonucleotide constructs. As explained for Figures 1 and 2, this requires addition of a reverse transcriptase together with additional elements required for synthesis of the first strand 118 such as nucleotides.

In step S306 the biological sample is scanned with focused light beams. In particular, a first region of interest is scanned with a first light beam and a second region of interest is scanned with a second light beam. The regions of interest are generally separate areas of the biological sample. Alternatively, the regions of interest may at least partially overlap. The first light beam and the second light beam have differing parameters, as described above. This means, for example, that the first and the second light beam each have a different wavelength. Thus, the first region of interest is scanned with the first light beam at a certain wavelength and the second region of interest is scanned with the second light beam at a different wavelength. The characteristic of the light beams, such as the wavelength, is chosen such that the first light beam removes the first cage molecule of the first plurality of oligonucleotide constructs and that the second beam removes the second cage molecule of the second plurality of oligonucleotide constructs. Thus, after scanning, the first region of interest contains uncaged oligonucleotide constructs originating from the first plurality of oligonucleotide constructs and the second region of interest contains uncaged oligonucleotide constructs originating from the second plurality of oligonucleotide constructs.

The regions of interest may be identified in the biological sample by staining or marking the biological structures of the biological sample with specific fluorescent markers and imaging the biological sample. Alternatively, the biological sample may be one of a plurality of associated tissue sections and one of the sections adjacent to the biological sample is stained and imaged. The region of interest may then be identified in the image data of the biological sample and coordinates of the identified regions of interest may be generated in order to direct the scanning light beam to the coordinates of the identified regions.

In step S308 the complementary second strand 122 is synthesised from the respective first or second plurality of oligonucleotide constructs. As explained for Figures 1 and 2, this requires addition of a DNA polymerase together with additional elements required for synthesis of the first strand 122 such as nucleotides. This generates first amplification templates from the uncaged oligonucleotide constructs originating from the first plurality of oligonucleotide constructs and second amplification templates from the uncaged oligonucleotide constructs originating from the second plurality of oligonucleotide constructs. The amplification templates may be used for further analysis or processing with molecular biology techniques. The method ends in step S310.

The first and second amplification templates may be amplified and sequenced, as described above for Figures 1 and 2. This requires lysis of the biological sample and extraction of the amplification templates from the biological sample. The sequence of the amplification templates may then be assigned to a region of interest by the sequence of their respective identifying region. Since the regions of interest were either scanned with the first or second light beam, only the first or second plurality of oligonucleotides were uncaged in the respective region of interest. The uncaged oligonucleotide constructs are therefore either from the first or second region of interest. The identifying region of the respective oligonucleotide construct enables identifying the sequence as being either from either the first or second plurality of oligonucleotide constructs.

Alternatively, the first and second oligonucleotide constructs may be extracted from the sample after a different step, for example, after synthesising the first strand 118.

Alternatively, in case the first and the second plurality of oligonucleotide constructs comprise a caged affinity reagent 202, the first and second amplification templates may be extracted from the biological sample and separated based on the affinity of the first and second affinity reagents.

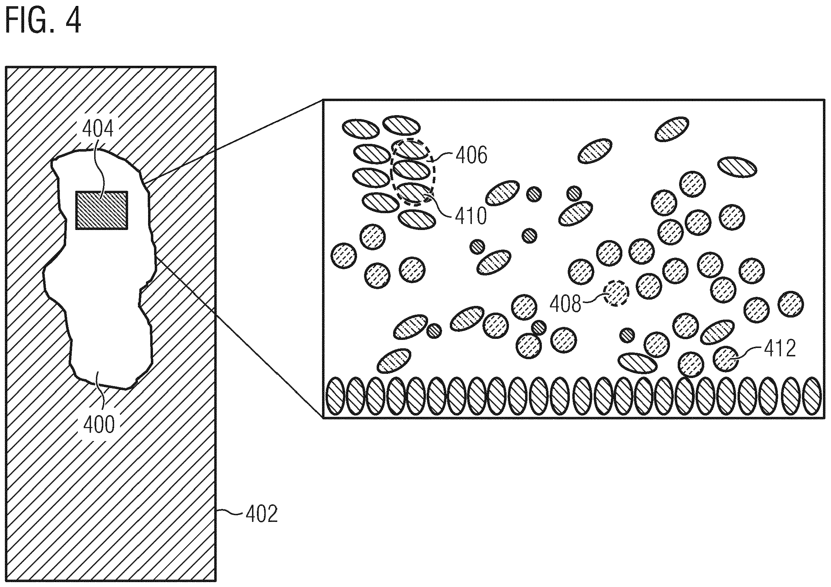

Figure 4 shows a schematic view of a biological sample 400 mounted on a glass slide 402. The inset shows a magnified view of a part 404 of the biological sample 402 with a first region of interest 406 and a second region of interest 408. The regions of interest 406, 408 are separate. Alternatively, the regions of interest 406, 408 may at least partially overlap. Each region of interest 406, 408 may comprise a single or a several biological structures of interest 410, 412 such as cells, tissue types or organelles. The regions of interest 406, 408 may in particular comprise cells of a single type, such as immune cells, cancerous cells or other functionally similar structures.

The glass slide 402 may be mounted on an imaging system comprising an imaging unit for imaging the biological sample and a scanning unit for scanning the first and second regions of interest with the first and second light beam, respectively. The imaging unit may image the biological sample initially in order to identify the regions of interest in the biological sample and generate coordinates that describe the position of the regions or interest within the biological sample, and/or on the glass slide 402, for example. The scanning unit may then scan the regions of interest defined by the coordinate system. The scanning system may comprise a light source such as a LED light source, to generate the focused light beams. Furthermore, the scanning unit may comprise two prisms arranged in the beam path of the first light beam and the second light beam in order to direct the light beams to the coordinates of the regions of interest. Specifically, the scanning unit comprises two prisms or wedge plates which are inclined against the optical axis of the light beams and can be rotated independently of each other about the optical axis. The document DE 100 18 253 C2 discloses a suitable arrangement and control of prisms or wedge plates. This enables moving the light beam with high spatial resolution by moving the prisms relative to each other, rather than moving the sample relative to the light beam.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100, 200: Oligonucleotide construct
- 102, 204: Amplification template
- 104: Promoter region
- 106: Identifying region
- 108: Adapter region
- 110: Target binding region
- 112: Photoremovable cage molecule
- 114: Poly-A tail
- 116: mRNA
- 118: First strand
- 120: Uncaged oligonucleotide construct
- 122: Second strand
- 202: Affinity reagent
- 400: Biological sample
- 402: Glass slide
- 404: Part of biological sample
- 406, 408: Region of interest
- 410, 412: Biological structure of interest

## Claims

1. A method for generating region-specific amplification templates (102, 204) of a biological sample (400), comprising the following steps:
adding at least a first plurality of oligonucleotide constructs (100, 200) and a second plurality of oligonucleotide constructs (100, 200) to the biological sample (400), wherein the oligonucleotide constructs (100, 200) each comprise a promoter region (104), an adapter region (108), a target binding region (110), wherein the first oligonucleotide constructs (100, 200) each further comprise a first identifying region (106) and a first photoremovable cage molecule (112), and wherein the second oligonucleotide constructs (100, 200) each further comprise a second identifying region (106) and a second photoremovable cage molecule (112),
synthesising a complementary first strand (118) from a template (116) bound to the target binding regions (110) of the oligonucleotide constructs (100, 200),
scanning at least a first region of interest (406, 408) of the biological sample (400) with a first focused light beam and a second region of interest (406, 408) of the biological sample (400) with a second focused light beam to form uncaged first oligonucleotide constructs (120) in the first region of interest (406, 408) and uncaged second oligonucleotide constructs (120) in the second region of interest (406, 408),
synthesising a complementary second strand (122) to form first amplification templates (102, 204) originating from the first region of interest (406, 408) and second amplification templates (102, 204) originating from the second region of interest (406, 408).

2. The method according to claim 1, wherein the first light beam and the second light beam have differing light parameters

3. The method according to one of the preceding claims, wherein the first cage molecule (112) is photoremovable from the first oligonucleotide construct (100, 200) only by the first focused light beam, and the second cage molecule (112) is photoremovable from the second oligonucleotide construct (100, 200) only by the second focused light beam.

4. The method according to one of the preceding claims, wherein the uncaged oligonucleotide constructs (120), or the amplification templates (102, 204) are extracted from the biological sample (400).

5. The method according to one of the preceding claims, wherein the first oligonucleotide construct (100, 200) further comprises a first affinity reagent (202) and the second oligonucleotide construct (100, 200) further comprises a second affinity reagent (202).

6. The method according to claim 5, wherein the first photoremovable cage molecule (112) is bound to the first affinity reagent (202) and the second photoremovable cage molecule (112) is bound to the second affinity reagent (202).

7. The method according to one of the claims 5 to 6, wherein the uncaged oligonucleotide construct (120) or the amplification templates (102, 204) are extracted selectively by affinity capture based on their respective affinity reagent (202).

8. The method according to one of the preceding claims, wherein the target binding region (110) is a poly-T region.

9. The method according to one of the preceding claims, wherein the amplification templates (102, 204) are amplified with a polymerase specific to the promoter regions (104) of the oligonucleotide constructs (100, 200).

10. The method according to claim 9, wherein the promoter region is a T7 promoter and the polymerase is a T7 polymerase.

11. The method according to one of the claims 9 to 10, wherein amplified amplification templates are sequenced.

12. The method according to one of the preceding claims, wherein the regions of interest (406, 408) are scanned by means of an optical scanning unit comprising two prisms arranged in the beam path of the first light beam and the second light beam.

13. The method according to one of the preceding claims, wherein the biological sample (400) is stained and imaged prior to directing the light beams to the regions of interest (406, 408), or
wherein the biological sample (400) is one of a plurality of sections and wherein one of the sections adjacent to the biological sample (400) is stained and imaged prior to directing the light beams to the region of interests.

14. The method according to claim 13, wherein at least the first and the second regions of interest (406, 408) of the biological sample (400) are identified in the image data and respective coordinates of a scanning coordinate system are generated to scan the respective coordinates of at least the first and second regions of interest (406, 408) with the respective light beam.

15. A system for performing the method according to one of the preceding claims, the system comprising:
an imaging unit configured to image a biological sample (400),
a scanning unit configured to scan at least a first and a second region of interest (406, 408) the biological sample (400) with a respective first or second focused light beam.

16. The system according to claim 15, wherein the scanning unit is configured to generate the first light beam and the second light beam with differing light parameters.

17. The system according to one of the claims 15 to 16, wherein the scanning unit comprises two prisms arranged in the beam path of the first and second light beam.
